# EUROPEAN PATENT SPECIFICATION

(11) **EP 1 644 371 B1**
(45) Date of publication and mention of the grant of the patent: **13.02.2008**
(21) Application number: 03742433.0
(22) Date of filing: 03.07.2003
(51) Int. Cl.: C07D 471/04

(54) **PYRAZOLOISOQUINOLINE DERIVATIVES AS KINASE INHIBITORS**
PYRAZOLOISOQUINOLINENDERIVATEN ALS KINASE INHIBITOREN
DERIVES DE PYRAZOLOISOQUINOLINE UTILES COMME INHIBITEURS DE KINASE

(43) Date of publication of application: 12.04.2006
(73) Proprietor: Aventis Pharmaceuticals Inc., Bridgewater, New Jersey 08807 (US)
(72) Inventor: MAJID, Tahir, N., Hoboken, NJ 07030 (US); HOPKINS, Corey, Hillsborough, NJ 08844 (US); PEDGRIFT, Brian, L., Flemington, NJ 08822 (US); COLLAR, Nicola, Jersey City, NJ 07310 (US); WIRTZ-BRUGGER, Friederike, Branchburg, NJ 08876 (US); MERRILL, Jean, Whippany, NJ 07981 (US)
(74) Representative: Then, Johann
(86) International application number: PCT/US2003/021144
(87) International publication number: WO 2005/012301

(56) References cited:
- WO-A-03/024936
- GB-A- 2 185 255
- US-A- 4 748 246
- YAMAMOTO YUMI ET AL: "Role of the NF-kappaB pathway in the pathogenesis of human disease states" CURRENT MOLECULAR MEDICINE, BENTHAM SCIENCE PUBLISHERS, GB, vol. 1, no. 3, July 2001 (2001-07), pages 287-296, XP001094465 ISSN: 1566-5240
- CECCHI, LUCIA ET AL: "Synthesis of 1,5-diaryl-3-methyl-1H-pyrazolo[4,5-c]isoq uinolines and studies of binding to specific peripheral benzodiazepine binding sites" JOURNAL OF PHARMACEUTICAL SCIENCES (1989), 78(6), 437-42 , XP000867420

## Description

The invention relates to novel pyrazoloisoquinoline derivatives, to processes for preparing them and to their use as pharmaceuticals, in particular their use for inhibiting kinases. More particularly, their use for inhibiting NFκB-inducing kinase (NIK). The pyrazoloisoquinoline derivatives can therefore be used for treating a variety of disease conditions that involve inflammatory component due to increase in NIK activity, particularly multiple sclerosis (MS).

NFκB is a heterodimeric transcription factor which is able to activate a large number of genes which encode, inter alia, proinflammatory cytokines such as IL-1, IL-2, TNFα or IL-6. NFκB is present in the cytosol of cells, where it is complexed with its naturally occurring inhibitor IκB. Stimulation of the cells, for example by cytokines, leads to the IκB being phosphorylated and subsequently broken down proteolytically. This proteolytic breakdown leads to the activation of NFκB, which then migrates into the nucleus of the cell, where it activates a large number of proinflammatory genes.

In diseases such as rheumatoid arthritis (in connection with inflammation), osteoarthritis or asthma, NFκB is activated beyond the normal extent. It has been demonstrated that pharmaceuticals such as glucocorticoids, salicylates or gold salts, which are used in the therapy of rheumatism, inhibit the NFκB-activating signal chain at various points or interfere directly with the transcription of the genes.

IkB kinase (IKK) has a central function in the NFkB signal transduction pathway since it mediates the phosphorylation of IkB. IKK is likewise activated by phosphorylation. The NFkB-inducing kinase (NIK) is a Ser/Thr kinase and participates in the activation of IKK. By means of overexpressing NIK in cell culture, it was possible to augment, in a stimulus-independent manner, the expression of NFkB-activated reporter genes or the expression of the NFkB-induced adhesion molecule ICAM1. NIK mediates this effect by interacting with, and phosphorylating, the IKKα sub-unit of IKK. By contrast, it was possible to inhibit the expression of an NFkB-activated reporter gene, and the IL1-induced expression of the adhesion molecule ICAM1, by overexpressing a dominant negative NIK mutant in cell culture. It was possible, by overexpressing the NIK C-terminal domain, which is responsible for interacting with IKK, to inhibit the TNFα-induced expression of an NFkB-activated reporter gene in cell culture. Pyrazoloisoquinoline derivatives which possess inhibitory activity directed against NIK are likewise able to inhibit the release of TNFα in LPS-stimulated and IL1β-stimulated human peripheral blood lymphocytes as well as the release of IL1β, TNFα and IL6 in LPS-stimulated whole human blood. Pyrazoloisoquinoline compounds possessing anti-inflammatory activity have already been described in the published document GB 2 185 255 A.

In the endeavor to obtain effective compounds for treating diseases whose course involves an increased activity of NFkB-inducing kinase, it has now been found that the pyrazoloisoquinoline derivatives according to the invention are strong and very specific inhibitors of NIK and exhibit good solubility in water.

The invention therefore relates to the compounds of the formula I 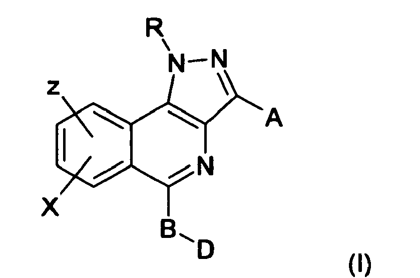 selected from the group consisting of:
5-pyridin-2-yl-3-trifluoromethyl-1H-pyrazolo[4,3-c]isoquinoline,
3-methyl-5-(2-trifluoromethyl-phenyl)-1H-pyrazolo[4,3-c]isoquinoline,
3-methyl-5-(3-trifluoromethyl-phenyl)-1H-pyrazolo[4,3-c]isoquinoline,
3-methyl-5-(4-trifluoromethyl-phenyl)-1H-pyrazolo[4,3-c]isoquinoline,
1,3-dimethyl-5-(3-trifluoromethylphenyl)-1H-pyrazolo[4,3-c]-isoquinoline,
1,3-dimethyl-5-(2,6-difluorophenyl)-1H-pyrazolo[4,3-c]-isoquinoline,
5-methoxymethyl-3-methyl-1H-pyrazolo[4,3-c]-isoquinoline,
7-methoxycarbonyl-3-methyl-5-phenyl-1H-pyrazolo[4,3-c]-isoquinoline,
7-methoxycarbonyl-3-methyl-5-pyridin-2-yl-1H-pyrazolo[4,3-c]-isoquinoline,
7-dimethylamino-3-methyl-5-phenyl-1H-pyrazolo[4,3-c]isoquinoline,
7-dimethylamino-3-methyl-5-pyridin-2-yl-1H-pyrazolo[4,3-c]-isoquinoline,
1, 3-dimethyl-5-(3-methyl-thiophen-2-yl)-1H-pyrazolo[4,3-c]-isoquinoline,
3-methyl-5-phenyl-9-trifluoromethyl-1H-pyrazolo[4,3-c]isoquinoline,
3-methyl-5-pyridin-2-yl-9-trifluoromethyl-1H-pyrazolo[4,3-c]-isoquinoline, and
3-methyl-5-(2,3,4,5,6-pentafluoro-phenyl)-1H-pyrazolo[4,3-c]-isoquinoline.

The invention also relates to a pharmaceutical composition, which is characterized by an effective content of at least one compound of the formula I selected from the above-identified group together with a pharmaceutically suitable and physiologically tolerated carrier substance.

On account of their pharmacological properties, the compounds according to the invention are suitable for producing a pharmaceutical for the selective prophylaxis and therapy of all those diseases whose course involves an increased activity of NIK.

The present invention also relates to the use of a compound selected from the above-identified group for producing a pharmaceutical for treating a disease condition selected from the group consisting of osteoarthritis, rheumatoid arthritis and asthma.

The pharmaceuticals according to the invention can be administered orally, by inhalation, rectally or transdermally or by means of subcutaneous, intraarticular, intraperitoneal or intravenous injection. Oral administration is preferred.

Examples of suitable solid or galenic preparation forms are granules, powders, sugar-coated tablets, tablets, (micro)capsules, suppositories, syrups, juices, suspensions, emulsions, drops or injectable solutions, and also preparations with protracted active compound release, in the preparation of which customary auxiliary substances, such as carrier substances, disintegrants, binders, coating agents, swelling agents, glidants or lubricants, flavorings, sweeteners and solubilizers are used. Frequently employed auxiliary substances which may be mentioned are magnesium carbonate, titanium dioxide, lactose, mannitol and other sugars, talc, milk protein, gelatin, starch, cellulose and its derivatives, animal and vegetable oils, such as cod liver oil, sunflower oil, groundnut oil or sesame oil, polyethylene glycol and solvents, such as sterile water and monohydric or polyhydric alcohols, such as glycerol.

The pharmaceutical preparations are preferably produced and administered in dosage units, with each unit containing as the active constituent, a particular dose of the compound of the formula II according to the invention. In the case of solid dosage units, such as tablets, capsules, sugar-coated tablets or suppositories, this dose can be up to about 1000 mg, preferably from about 50 mg to 300 mg, and, in the case of injection solutions in ampoule form, up to about 300 mg, preferably from about 10 mg to 100 mg.

Depending on the activity of the compound according to selected from the above-identified group, daily doses of from about 20 mg to 1000 mg of active compound, preferably of from about 100 mg to 500 mg, are indicated for treating an adult patient of about 70 kg in weight. However, higher or lower daily doses may also possibly be appropriate. The daily dose can be administered either by means of a once-only administration in the form of a single dosage unit, or of several smaller dosage units, or by means of the multiple administration of subdivided doses at predetermined intervals.

As a rule, mass-spectroscopic methods (FAB-MS, ESI-MS) are used for determining end products, the main peak being given in each case. Temperatures are given in degrees centigrade; RT denotes room temperature (from 22°C to 26°C). Abbreviations which are used are either explained or correspond to the customary conventions.

The invention is explained in more detail below with the aid of examples.

### Preparation examples

In general the compounds of this invention as prepared below were characterized by High Pressure Liquid Chromatograph (HPLC) using one of the following three methods:
Method A: HPLC was run on Synergi 2U Hydro-RP 20X4.0MM COL, water (0.1 % trifluoroacetic acid/acetonitrile (0.1 % trifluoroacetic acid) = 10/90 → 90/10).
Method B: Agilent 1100 Series LC/MSD YMC Pro C₁₈ S5 120A 4.6X30MM Col, Waterf (0.1 % trifluoroacetic acid/acetonitrile (0.1 % trifluoroacetic acid) = 95/5 → 5/95), run time 5 min.
Mass S[ectra were run as follows:
Method A: Micromass LCT-TOF MS, Scan M/Z 100-1000.
Method B: Micromass LCZ-TOF MS, Scan M/Z 100-800.

### Reference Example 1

### 3,5-Diphenyl-1H-pyrazolo[4,3-c]isoquinoline (1)

### a) N-(3,5-diphenyl-1H-pyrazol-4-yl)benzylamine (2)

574 mg of hydroxybenzotriazole and 822 µl of diisopropylcarbodiimide were added to a solution of 260 mg of benzoic acid in 10 ml of methylene chloride, then 500 mg of 3,5-diphenyl-1H-pyrazol-4-ylamine in 2 ml of acetonitrile were added dropwise, at 0 °C; the mixture was then stirred at room temperature (RT) for 12 hours (h), after which water was added and the whole was extracted with ethyl acetate. The organic phase was dried over magnesium sulfate and concentrated under reduced pressure. The residue contained the title compound and was used in the following reaction without further purification.

### b) 3,5-Diphenyl-1H-pyrazolo[4,3-c]isoquinoline (1)

201 mg of phosphorus pentoxide were added to a solution of 240 mg of N-(3,5-diphenyl-1H-pyrazol-4-yl)benzylamine (2) in 10 ml of xylene, after which 195 µl of phosphorus oxychloride were added dropwise at 150°C. The reaction solution was stirred at 150°C for 4 h and then stirred at RT for 12 h; a saturated solution of sodium hydrogen carbonate was then added and the whole was extracted with methylene chloride. The organic phase was dried over magnesium sulfate, concentrated under reduced pressure and purified by means of HPLC (Merk-Hibar-Lichrospher 100-RP-18, water (0. 1 % trifluoroacetic acid)/acetonitrile (0.1 % trifluoroacetic acid) = 80/20 → 10/90). The following was obtained:
1. C₂₂H₁₅N₃ (321.38); MS (ESI) 322 (M+H)

### Reference Example 2

### 5-(3-Methoxyphenyl)-3-methyl-1H-pyrazolo[4,3-c]isoquinoline (3)

### a) 3-Methoxy-N-(3-methyl-5-phenyl-1H-pyrazol-4-yl)benzamide (4)

311 mg of hydroxybenzotriazole and 445 µl of diisopropylcarbodiimide were added to a solution of 160 mg of 3-methoxybenzoic acid in 10 ml of dimethylformamide, after which 200 mg of 3-methyl-5-phenyl-1H-pyrazol-4-ylamine were added; the mixture was then stirred at RT for 12 h, after which water was added and the whole was extracted with methylene chloride. The organic phase was dried over magnesium sulfate, concentrated under reduced pressure and purified by means of HPLC (Merk-Hibar-Lichrospher 100-RP-18, water (0.1 % trifluoroacetic acid)/acetonitrile (0.1 % trifluoroacetic acid) = 80/20 → 10/90). The following was obtained: 4 C₁₇H₁₅N₃O₂ (293.33); MS (ESI) 294 (M+H).

### b) 5-(3-Methoxyphenyl)-3-methyl-1H-pyrazolo[4,3-c]isoquinoline (3)

128 mg of 3-methoxy-N-(3-methyl-5-phenyl-1H-pyrazol-4-yl)benzamide (4) were suspended in 5 ml of xylene and the suspension was boiled at 160°C; 119 mg of phosphorus pentoxide were then added and the mixture was subsequently stirred at 160°C for 15 min. 27 µl of phosphorus oxychloride were added dropwise to the suspension, which was then stirred at 160°C for 12 h; a saturated solution of sodium hydrogen carbonate was then added and the whole was extracted with ethyl acetate. The organic phase was dried over magnesium sulfate, concentrated under reduced pressure and purified by means of HPLC (Merk-Hibar-Lichrospher 100-RP-18, water (0.1 % trifluoroacetic acid)/acetonitrile (0.1 % trifluoroacetic acid) = 80/20 → 10/90). The following was obtained: 3. C₁₈H₁₅N₃O (298.34); MS (ESI 290 (M+H)

### Reference Example 3

### 3-(3-Methyl-1H-pyrazolo[4,3-c]isoquinolin-5-yl)phenol (5)

380 µl of 1M boron tribromide solution in methylene chloride were added dropwise, at -78°C, to a solution of 55 mg of 5-(3-methoxyphenyl)-3-methyl-1H-pyrazolo[4,3-c]isoquinoline (3) in 3 ml of methylene chloride. The reaction solution was stirred at RT for 12 h, concentrated under reduced pressure and purified by means of HPLC (Merk-Hibar-Lichrospher 100-RP-18, water (0.1 % trifluoroacetic acid)/acetonitrile (0.1 % trifluoroacetic acid) = 80/20 → 10/90). The following was obtained: 5.
C₁₇H₁₃N₃O (275.31); MS (ESI 276 (M+H)
of 4-diethylamino-N-(3-methyl-5-phenyl-1H-pyrazol-4-yl)benzamide (27). The following was obtained: 26. C₂₁H₂₂N₄ (330.44);

### Reference Example 15

### 3-Methyl-5-pyridin-4-yl-1H-pyrazolo[4,3-c]isoquinoline (28)

### a) N-(3-Methyl-5-phenyl-1H-pyrazol-4-yl)isonicotinamide (29)

205 mg of isonicotinoyl chloride*HCl were added to a solution of 200 mg of 3-methyl-5-phenyl-1H-pyrazol-4-ylamine in 2 ml of pyridine and the mixture was then stirred at RT for 12 h; water was then added and the whole was extracted with methylene chloride. The organic phase was dried over magnesium sulfate, concentrated under reduced pressure and purified by means of HPLC (Merk-Hibar-Lichrospher 100-RP-18, water (0.1% trifluoroacetic acid)/acetonitrile (0.1% trifluoroacetic acid) = 80/20 → 10/90). The following was obtained: 27. C₁₆H₁₄N₄O (278.32); MS (ESI) 279 (M+H)

### b) 3-Methyl-5-pyridin-4-yl-1H-pyrazolo[4,3-c]isoquinoline (28)

The preparation took place in analogy with Example 1b), starting with 110 mg of N-(3-methyl-5-phenyl-1H-pyrazol-4-yl)isonicotinamide (29). The following was obtained: 28. C₁₆H₁₂N₄ (260.30); MS (ESI) 261 (M+H)

### Reference Example 21

### 7,8-Dimethoxy-3-methyl-5-phenyl-1H-pyrazolo[4,3-c]isoquinoline (40)

### a) 1-(3,4-Dimethoxyphenyl)butane-1,3-dione (41)

3.9 g of sodium hydride were initially introduced into 150 ml of cyclohexane, and a solution of 15 g of 1-(3,4-dimethoxyphenyl)ethanone in 16.3 ml of ethyl acetate was added. The reaction solution was boiled at 80°C for 1 h, after which acetic acid was added and the whole was extracted with MTB-ether. The MTB-ether phase was dried over magnesium sulfate and subjected to rotary evaporation. The residue was chromatographed through silica gel (ethyl acetate/heptane 1/6). The following was obtained: 41 C₁₂H₁₄O₄ (222.24); MS (ESI) 223 (M+H)

### b) 1-(3,4-Dimethoxyphenyl)butane-1,2,3-trione 2-oxime (42)

5 g of 1-(3,4-dimethoxyphenyl)butane-1,3-dione (41) were initially introduced into 25 ml of acetic acid, and 1.71 g of sodium nitrite, dissolved in 5 ml of water, were then added dropwise at 15°C. The reaction solution was stirred at RT for 1 h and then remained standing for 2 h. 50 g of ice were added to the solution and the whole was then stored at 0°C for 12 h, in connection with which the product precipitated out. The product was subsequently filtered off with suction and dried at 50°C in a drying oven. The following was obtained: 42 C₁₂H₁₃NO₅ (251.24); MS (ESI) 252 (M+H)

### c) 5-(3,4-Dimethoxyphenyl)-3-methyl-4-nitro-1H-pyrazole (43)

6.77 g of 1-(3,4-dimethoxyphenyl)butane-1,2,3-trione 2-oxime (42) were dissolved in 54 ml of acetic acid, after which 0.96 g of hydrazine was added dropwise at RT and the mixture was subsequently stirred at 60°C for 2 h. Ice was added to the reaction solution, which was then neutralized with sodium carbonate and extracted with MTB-ether. The organic phase was dried over magnesium sulfate and subjected to rotary evaporation. The following was obtained: 43. C₁₂H₁₃N₃O₃ (247.26);
MS (ESI) 248 (M+H)

### d) 5-(3,4-Dimethoxyphenyl)-3-methyl-1H-pyrazol-4-ylamine (44)

4.84 g of 5-(3,4-dimethoxyphenyl)-3-methyl-4-nitro-1H-pyrazole (43) were dissolved in 80 ml of ethanol, and 0.5 g of Pd/C was added to this solution. The solution was shaken under hydrogen for 2 h, then filtered through magnesium sulfate and subjected to rotary evaporation. The residue was chromatographed through silica gel (ethyl acetate/heptane 3/1). The following was obtained: 44 C₁₂H₁₅N₃O₂ (233.27); MS (ESI) 234 (M+H)

### e) N-[5-(3,4-Dimethoxyphenyl)-3-methyl-1H-pyrazol-4-yl]benzamide (45)

The preparation took place in analogy with Example 15a), starting with 500 mg of 5-(3,4-dimethoxyphenyl)-3-methyl-1H-pyrazol-4-ylamine (44) and 280 µl of benzoyl chloride. The following was obtained: 45
C₁₉H₁₉N₃O₃ (337.38); MS (ESI) 338 (M+H)

### f) 7,8-Dimethoxy-3-methyl-5-phenyl-1H-pyrazolo[4,3-c]isoquinoline (40)

The preparation place in analogy with Example 1b), starting with 187 mg of N-[5-(3,4-dimethoxyphenyl)-3-methyl-1H-pyrazol-4-yl]benzamide (45). The following was obtained: 40
C₁₉H₁₇N₃O₂ (319.37) MS (ESI) 320 (M+H)

### Reference Example 22

### 7-Methoxy-3-methyl-5-phenyl-1H-pyrazolo[4.3-c]isociuinoline (46)

### a) 1-(4-Methoxyphenyl)butane-1,3-dione (47)

The preparation took place in analogy with Example 21 a), starting with 15 g of 1-(4-methoxyphenyl)ethanone. The following was obtained: 47
C₁₁H₁₂O₃ (192.22); MS (ESI) 193 (M+H)

### b) 1-(4-Methoxyphenyl)butane-1,2,3-trione 2-oxime (48)

The preparation took place in analogy with Example 21b), starting with 5 g of 1-(4-methoxyphenyl)butane-1,3-dione (47). The following was obtained: 48 C₁₁H₁₁NO₄ (221.21 MS (ESI) 222 (M+H)

### c) 5-(4-Methoxyphenyl)-3-methyl-4-nitroso-1H-pyrazole (49)

The preparation took place in analogy with Example 21c), starting with 4.19 g of 1-(4-methoxyphenyl)butane-1,2,3-trione 2-oxime (48). The following was obtained: 49. C₁₁H₁₁N₃O₂ (217.23) MS (ESI) 218 (M+H)

### d) 5-(4-Methoxyphenyl)-3-methyl-1H-pyrazol-4-ylamine (50)

The preparation took place in analogy with. Example 21d), starting with 3.17 g of 5-(4-methoxyphenyl)-3-methyl-4-nitro-1H-pyrazole (49). The following was obtained: 50. C₁₁H₁₃N₃O (203.25) MS (ESI) 204 (M+H)

### e) N-[5-(4-Methoxyphenyl)-3-methyl-1H-pyrazol-4-yl]benzamide (51)

The preparation took place in analogy with Example 15a), starting with 500 mg of 5-(4-methoxyphenyl)-3-methyl-1H-pyrazol-4-ylamine (50) and 315 µl of benzoyl chloride. The following was obtained: 51
C₁₈H₁₇N₃O₂ (307.36) MS (ESI) 308 (M+H)

### 1) 7-Methoxy-3-methyl-5-phenyl-1H-pyrazolo[4,3-c]isoquinoline (46)

The preparation took place in analogy with Example 1b), starting with 230 mg of N-[5-(4-methoxyphenyl)-3-methyl-1H-pyrazol-4-yl]benzamide (51). The following was obtained: 46
C₁₈H₁₅N₃O (289.34) MS (ESI) 290 (M+H)

### Reference Example 22A

### 7-Methoxy-3-methyl-5-phenyl-1H-pyrazolo[4,3-c]isoquinoline (46)

The title compound was also prepared by a modified procedure as described herein using the procedures as set forth below.

### a) 5-(4-Methoxy-phenyl)-3-rnethyl-1H-pyrazol-4-ylamine (A2) (Example 22(d)

6.80 g of sodium dithionite was added to a mixture of 1.23 g of 5-(4-methoxyphenyl)-3-methyl-4-nitroso-1H-pyrazole (Example 22(c)) and 50 ml of water at 70°C, and the reaction mixture was stirred for 15 minutes. 5 ml of ethanol was added and the reaction mixture was stirred for 10 minutes. The reaction mixture was extracted with ethyl acetate. The organic layer was washed with brine, dried over magnesium sulfate, filtered and concentrated. Trituration with ether gave the title compound as a beige solid, melting point 120-122°C, C₁₁H₁₃N₃O (203.24), MS (ESI, method B) 204 (M+H).

### b) N-[5-(4-Methoxy-phenyl)-3-methyl-1H-pyrazol-4-yl]-benzamide (A1) (Example 22 (e))

110 µL of pyridine were added to a solution of 230 mg of 5-(4-methoxyphenyl)-3-methyl-1H-pyrazol-4-ylamine in 10 ml of dichloromethane, followed immediately by 174 mg of benzoyl chloride. The reaction mixture was stirred at room temperature for 2 h and then concentrated. The residue was purified by MPLC, eluting with 80/20 ethyl acetate/heptane to give the title compound as a white solid, melting point 230-232°C, C₁₈H₁₇N₃O₂ (307.36), MS (ESI, method B) 308 (M+H).

### c) 7-Methoxy-3-methyl-5-phenyl-1H-pyrazolo[4,3-c]isoquinoline (A) (Example 22(f))

740 mg of phosphorus pentoxide were added to a solution of 108 mg of N-[5-(4-Methoxy-phenyl)-3-methyl-1H-pyrazol-4-yl]-benzamide in 1.7 mL of phosphorus oxychloride and the reaction solution was stirred at reflux for 4.5 h. After cooling, ice was added carefully, followed by a saturated solution of sodium hydrogen carbonate. The whole was extracted with ethyl acetate. The organic phase was washed with brine, dried over sodium sulfate, concentrated under reduced pressure and the residue was purified by means of MPLC, eluting with 50/50 heptane/ethyl acetate. This gave the title compound as an off-white solid, C₁₈H₁₅N₃O (289.34), MS (ESI, method A) 290 (M+H), HPLC R_{T} 2.87 min.

### Example 25

### 7,8-Dimethoxy-3-methyl-5-pyridin-3-yl-1H-pyrazolo[4,3-c]isoquinoline (56)

### a) N-[5-(3,4-Dimethoxyphenyl)-3-methyl-1H-pyrazol-4-yl]nicotinamide (57)

The preparation took place in analogy with Example 15a), starting with 200 mg of 5-(3,4-dimethoxyphenyl)-3-methyl-1H-pyrazol-4-ylamine (44) and 168 mg of nicotinoyl chloride*HCl. The following was obtained: 57.
C₁₈H₁₈N₄O₃ (338.37) . MS(ESI) 339 (M+H)

### b) 7,8-Dimethoxy-3-methyl-5-pyridin-3-yl-1H-pyrazolo[4,3-c]isoquinoline (56)

The preparation took place in analogy with Example 1b), starting with 126 mg of N-[5-(3,4-dimethoxyphenyl)-3-methyl-1H-pyrazol-4-yl]nicotinamide (57). The following was obtained: 56
C₂₀H₁₉N₃O₃ (320.35) MS (ESI) 321 (M+H)

### Example 26

### 7-Methoxy-5-(3-methoxyphenyl)-3-methyl-1H-pyrazolo-[4,3-c]isoquinoline (58)

### a) 3-Methoxy-N-[5-(4-methoxyphenyl)-3-methyl-1H-pyrazol-4-yl]benzamide (59)

The preparation took place in analogy with Example 15a), starting with 300 mg of 5-(4-methoxyphenyl)-3-methyl-1H-pyrazol-4-ylamine (50) and 277 mg of 3-methoxybenzoyl chloride. The following was obtained: 59.
C₁₉H₁₉N₃O₃ (337.38) MS (ESI) 338 (M+H)

### b) 7-Methoxy-5-(3-methoxyphenyl)-3-methyl-1H-pyrazolo[4,3-c]isoquinoline (58)

The preparation took place in analogy with Example 1b), starting with 263 mg of 3-methoxy-N-[5-(4-methoxyphenyl)-3-methyl-1H-pyrazol-4-yl]benzamide (59). The following was obtained: 58
C₁₉H₁₇N₃O₂ (319.37) MS (ESI) 320 (M+H) .

### Example 27

### 5-Phenyl-1H pyrazolo[4,3-c]isoquinoline-3-carboxylic acid (60)→

2.1 g of potassium permanganate in 36 ml of water were added to a solution of 600 mg of 5-phenyl-3-methyl-1H-pyrazolo[4,3-c]isoquinoline in 36 ml of pyridine. The mixture was stirred at 40°C for 12 h. The resulting suspension was filtered with suction through silica gel, after which the filtrate was concentrated under reduced pressure and the residue was purified by means of HPLC (Merk-Hibar-Lichrospher 100-RP-18, water (0.1 % trifluoroacetic acid)/acetonitrile (0.1 % trifluoroacetic acid) = 80/20 → 10/90). The following was obtained: 60.
C₁₇H₁₁N₃O₂ (289.30) MS (ESI) 290 (M+H)

### Reference Example 33

### 3-Methyl-5-phenyl-1H-pyrazolo[4,3-c]isoquinoline (B).

### a) N-(3-Methyl-5-phenyl-1H-pyrazol-4-yl)-benzamide (B1).

To a rt solution of 550 mg of 3-methyl-5-phenyl-1H-pyrazol-4-ylamine (prepared in accordance with the procedure set forth in UK 2 185 255, Example 2A) in 8.5 mL pyridine was added 370 µL of benzoyl chloride. The reaction was stirred for 1 h and then concentrated under reduced pressure. The residue was dissolved in chloroform and washed with 0.5 N hydrochloric acid and then saturated aqueous sodium carbonate. The organic portion was dried with sodium sulfate and concentrated under reduced pressure and purified by means of MPLC (12 g SiO₂, 40% ethyl acetate:heptane) to give the title compound as a colorless solid.
HPLC R_{T} = 2.35 min.
C₁₇H₁₅N₃O (277.33) MS, (ESI, method A) 278 (M+H).

### b) 3-Methyl-5-phenyl-1H-pyrazolo[4,3-c]isoquinoline (B).

To a mixture of 565 mg of N-(3-Methyl-5-phenyl-1H-pyrazol-4-yl)-benzamide was added 5 mL of nitrobenzene followed by 270 µL of POCl₃ and the resulting mixture was heated at 185 °C. After 1 h, 0.4 mL of a SnCl₄ solution (1.0 M solution in heptane) was added and the mixture was heated for an additional 2 h, then concentrated under reduced pressure and purified by means of MPLC (12 g SiO₂, 35% ethyl acetate:heptane) to give the title compound as a tan solid.
HPLC R_{T} = 2.85 min.
C₁₇H₁₃N₃ (259.31) MS, (ESI, method A) 260 (M+H).

### Reference Example 34

### 3-Methyl-5-thiophen-2-yl-1H-pyrazolo[4.3-c]isoquinoline (D).

### a) Thiophene-2-carboxylic acid (3-methyl-5-phenyl-1H-pyrazol-4-yl)-amide (D1).

The preparation took place in analogy to Example 33(B1) starting with 120 µL of 2-thiophenecarbonyl chloride, except the product was purified using MPLC (12 g SiO₂, 30% ethyl acetate:heptane) to give the title compound as an amorphous, orange solid.
HPLC R_{T} = 2.28 min.
C₁₅H₁₃N₃OS (283.36) MS (ESI, method A) 284 (M+H).

### b) 3-Methyl-5-thiophen-2-yl-1H-pyrazolo[4,3-c]isoquinoline (D).

To a suspension of 682.4 mg of thiophene-2-carboxylic acid (3-methyl-5-phenyl-1H-pyrazol-4-yl)-amide in 11.0 mL of phosphorous oxychloride was added 5.00 g of phosphorous pentoxide and the resulting suspension was heated at 120°C for 5 h. The reaction mixture was cooled to rt and quenched with the addition of ice and water and then neutralized with solid sodium carbonate. The aqueous mixture was extracted with ethyl acetate and the combined organic extracts were washed with brine, dried (magnesium sulfate) and concentrated under reduced pressure. The residue was purified by means of MPLC (4 g SiO₂, 30% ethyl acetate:dichloromethane) to give the title compound as a tan solid.
HPLC R_{T} = 3.19 min.
C₁₅H₁₁N₃S (265.34) MS (ESI, method A) 266 (M+H).

### Reference Example 39

### 5-Benzo[b]thiophene-2-yl-3-methyl-1H-pyrazolo[4,3-c]isoquinoline.

### a) Benzo[b]thiophene-2-carboxylic acid (3-methyl-5-phenyl-1H-pyrazol-4-yl)-amide (11).

The preparation took place in analogt to Example 33 (B1) starting with 380 mg of 3-methyl-5-phenyl-1H-pyrazol-4-ylamine in 4.6 mL pyridine and 454 mg benzo[b]thiophene-2-carbonyl chloride. The residue after work-up contained the title compound as a yellow solid and was used without further purification.
HPLC R_{T} = 2.77 min.
C₁₉H₁₅N₃OS (333.41) MS (ESI, method A) 334 (M+H).

### b) 5-Benzo[b]thiophene-2-yl-3-methyl-1H-pyrazolo[4,3-c]isoquinoline (I).

The preparation took place in analogy to Example 34 (D) starting with 544 mg of benzo[b]thiophene-2-carboxylic acid (3-methyl-5-phenyl-1H-pyrazol-4-yl)-amide, except the residue was purified by being recrystallisation from ethyl acetate to give the title compound as a tan solid.
HPLC R_{T} = 3.62 min.
C₁₉H₁₃N₃S (315.40) MS (ESI, method A) 316 (M+H).

### Reference Example 44

### 3-Methyl-5-(3-methyl-thiophen-2-yl)-1H-pyrazolo[4.3-c]isoquinoline.

### a) 3-Methyl-thiophene-2-carboxylic acid (3-methyl-5-phenyl-1H-pyrazol-4-yl)-amide (N1).

The preparation took place in analogy to Example 39 (11) starting with 414 mg of 3-methylthiophene-2-carbonyl chloride. The title compound was obtained as a tan solid.
HPLC R_{T} = 2.45 min.
C₁₅H₁₆N₃OS (297.39) MS (ESI, method A) 298 (M+H).

### b) 3-Methyl-5-(3-methyl-thiophen-2-yl)-1H-pyrazolo[4,3-c]isoquinoline (N).

The preparation took place in analogy to Example 34 (D) starting with 562 mg of 3-methyl-thiophene-2-carboxylic acid (3-methyl-5-phenyt-1H-pyrazol-4-yl)-amide, except the residue was dissolved in methanol and then purified by MPLC (4 g SiO₂, 10% ethyl acetate:dichloromethane, more polar fraction) to yield the title compound as a tan solid, along with the N-methylated compound (see, Example 45 (O) below).
HPLC R_{T} = 2.95 min.
C₁₆H₁₃N₃S (279.08) MS (ESI, method A) 280 (M+H).

### Example 45

### 1.3-Dimethyl-5-(3-methyl-thiophen-2-yl)-1H-pyrazolo[4,3-c]isoquinoline.

### a) 1,3-Dimethyl-5-(3-methyl-thiophen-2-yl)-1H-pyrazolo[4,3-c]isoquinoline (O).

The compound was isolated along with 3-methyl-5-(3-methyl-thiophen-2-yl)-1H-pyrazolo[4,3-c]isoquinoline (compound N of Example 44 above) by means of MPLC (4 g SiO₂, 10% ethyl acetate:dichloromethane, less polar fraction) to yield the title compound as a tan solid.
HPLC R_{T} = 3.25 min.
C₁₇H₁₅N₃S (293.10) MS (ESI, method A) 294 (M+H).

### Reference Example 49

### 5-(2-Chloro-phenyl)-3-methyl-1H-pyrazolo[4,3-c]isoquinoline

### a) 2-Chloro-N-(3-methyl-5-phenyl-1H-pyrazol-4-yl)-benzamide (S1)

The preparation took place in analogy to Example 33 (B1), starting with 266 mg of 2-chloro-benzoylchloride, except the reaction mixture was stirred for 24 h before concentrating. Trituration of the reside with dichloromethane gave the title compound as a white precipitate.
HPLC R_{T} 2.42 minutes
C₁₇H₁₄ClN₃O (312.06) MS (ESI, method A) 313 (M+H)

### b) 5-(2-chloro-phenyl)-3-methyl-1H-pyrazolo[4,3-c]isoquinoline (S)

The preparation took place in analogy to Example 33 (B), using 401 mg of 2-chloro-N-(3-methyl-5-phenyl-1H-pyrazol-4-yl)-benzamide. The reaction mixture was diluted with water and extracted with dichloromethane. The organic portion was concentrated under reduced pressure and purified by MPLC (4g SiO₂, dichloromethane/methanol =99/1 → 50/50) to give the title compound.
HPLC R_{T} 2.94 minutes

### Reference Example 50

### 5-(4-Fluoro-phenyl)-3-methyl-1H-pyrazolo[4,3-c]lisoquinoline

### a) 4-Fluoro-N-(3-methyl-5-phenyl-1H-pyrazol-4-yl)-benzamide (T1)

The preparation took place in analogy to Example 49 (S1), starting with 241 mg of 4-fluorobenzoyl chloride to give the title compound as a white precipitate.
HPLC R_{T} 2.46 minutes.
C₁₇H₁₄FNO₃ (295.31) MS (ESI, method A) 296 (M+H)

### b) 5-(4-Fluoro-phenyl)-3-methyl-1H-pyrazolo[4,3-c]isoquinoline (T)

The preparation was carried out as in Example 22A using 236 mg of 4-fluoro-N-(3-methyl-5-phenyl-1H-pyrazol-4-yl)-benzamide, except the reaction mixture was refluxed for 10 h. The cream solid residue after workup was the title compound.
HPLC (Method A) R_{T}
C₁₇H₁₂FN₃ (297.30) MS (ESI, )

### Example 59

### 3-Methyl-5-(2,3,4,5,6-pentafluoro-phenyl)-1H-pyrazolo[4,3-c]isoquinoline

### a) 2,3,4,5,6-Pentafluoro-N-(3-methyl-5-phenyl-1H-pyrazol-4-yl)-benzamide (AC1)

The preparation took place in analogy to example 33 (B1) using 400 mg of 2,3,4,5,6-pentafluoro-benzoyl chloride. The residue was triturated with methanol/dichloromethane (95/5) to give the title compound.
H PLC R_{T} 2.72 min
C₁₇H₁₀ F₅N₃O (367.28) MS (ESI, method A) 368(M+H)

### b) 3-Methyl-5-(2,3,4,5,6-pentafluoro-phenyl)-1H-pyrazolo[4,3-c]isoquinoline (AC)

The preparation was carried out as in Example 50 (T) using 147 mg of 2,3,4,5,6-pentafluoro-N-(3-methyl-5-phenyl-1H-pyrazol-4-yl)-benzamide, to give the title compound as a pale cream solid.
HPLC (Method A) R_{T} 3.15 min
C₁₇H₈F₅N₃ (349.06) MS (ESI, Method A) 350 (M+H)

### Example 69

### 5-Methoxymethyl-3-methyl-1H-pyrazolo[4,3-c]isoquinoline.

### a) 2-Methoxy-N-(3-methyl-5-phenyl-1H-pyrazol-4-yl)-acetamide (BA1).

To a rt solution of 416 mg of 3-methyl-5-phenyl-1H-pyrazol-4-ylamine in 4.6 mL pyridine was added 242 µL of methoxyacetyl chloride. After 2 h at rt, the reaction mixture was concentrated under reduced pressure and the residue was dissolved in chloroform and washed with 0.5 N hydrochloric acid and saturated aqueous sodium carbonate and brine. The organic portion was dried with sodium sulfate and concentrated under reduced pressure. The residue contained the title compound as a tan, amourphous solid and was used without further purification.
HPLC R_{T} = 1.80 min.
C₁₃H₁₅N₃O₂ (245.29) MS (ESI) 246 (M+H).

### b) 5-Methoxymethyl-3-methyl-1H-pyrazolo[4,3-c]isoquinoline (BA).

To a suspension of 320 mg of 2-methoxy-N-(3-methyl-5-phenyl-1H-pyrazol-4-yl)-acetamide in 6 mL of phosphorous oxychloride was added 2.70 g of phosphorous pentoxide and the resulting suspension was heated at 120 °C for 5 h. The reaction mixture was cooled to rt and quenched with the addition of ice and water and then neutralized with solid sodium carbonate. The aqueous mixture was extracted with ethyl acetate and the combined organic extracts were washed with brine, dried (magnesium sulfate) and concentrated under reduced pressure. The residue was purified recrystallisation from ethyl acetate. The title compound was obtained as a reddish, tan solid.
HPLC R_{T} = 2.42 min.
C₁₃H₁₃N₃O (227.27) MS (ESI) 228 (M+H).

### Example 72

### 3-Methyl-5-(4-trifluoromethyl-phenyl)-1H-pyrazole[4,3-c]isoquinoline

### a) N-(3-methyl-5-phenyl-1H-pyrazole-4-yl)-4-trifluoromethyl-benzamide (BD1)

361 mg of 4-trifluoromethylbenzoyl chloride were added dropwise to a solution of 300 mg of 3-methyl-5-phenyl-1H-pyrazol-4-ylamine in 5ml of pyridine at room temperature. The reaction mixture was stirred for 16 h and then concentrated. The residue was extracted into dichloromethane and washed with 0.5M hydrochloric acid, at which point the title compound precipitated.
HPLC R_{T} 2.77 min
C₁₈H₁₄F₃N₃O 345.32MS (ESI, method A) 346 (M+H)

### b) 3-Methyl-5-(4-trifluoromethyl-phenyl)-1H-pyrazole[4, 3-c]isoquinoline (BD)

A solution of 393 mg of N-(3-methyl-5-phenyl-1H-pyrazole-4-yl)-4-trifluoromethyl-benzamide in 15ml of phosphorus-oxychloride was treated with 2.557g of phosphorus pentoxide and stirred at 120°C for 32 hours. The reaction mixture was concentrated and the residue poured carefully onto ice water. The mixture was neutralized with sodium hydrogen carbonate and extracted with ethyl acetate. The organic portion was dried over magnesium sulfate, filtered and concentrated to give a solid. Purification by MPLC (5g SiO₂, ethyl acetate/heptane, gradient of 10/90→ 100/0) gave the title compound.
HPLC R_{T} 3.54 minutes.
C₁₈H₁₂F₃N₃ (327.31) MS (ESI, method A) 328 (M+H)

### Example 73

### 3-Methyl-5-(2-trifluoromethyl-phenyl)-1H-pyrazole[4,3-c]isoquinoline

### a) N-(3-Methyl-5-phenyl-1H-pyrazol-4-yl)-2-trifluoromethyl-benzamide (BE1)

The preparation took place in analogy with Example 72 (BD1), using 317 mg of 2-trifluoromethyl-benzoyl chloride, except the title compound was isolated by filtration directly from the reaction mixture.
HPLC R_{T} 2.54 minutes
C₁₈H₁₄F₃N₃O (345.33) MS (ESI, method A) 346 (M+1)

### b) 3-Methyl-5-(2-trifluoromethyl-phenyl)-1H-pyrazole[4, 3-c]isoquinoline (BE)

A solution of 174 mg of N-(3-methyl-5-phenyl-1H-pyrazole-4-yl)-2-trifluoromethyl-benzamide and 67 µl of phosphorus oxychloride in 2ml of nitrobenzene was heated at 185°C. After 1 h 118 µl of SnCl₄ solution (1.0M in heptane) was added and the reaction heated for a further 2 hours. The reaction mixture was concentrated and purified by MPLC (4g SiO₂, ethyl acetate/heptane, gradient of 50/50 →100/0, then ethyl acetate/ methanol, gradient of 95/5→80/20) to give the title compound.
HPLC R_{T} 2.92 minutes
C₁₈H₁₂F₃N₃ (327.31) MS (ESI, method A) 328 (M+1)

### Example 74

### 3-Methyl-5-(3-trifluoromethyl-phenyl)-1H-pyrazolo[4,3-c]isoquinoline, trifluoroacetic acid salt

### a) N-(3-Methyl-5-phenyl-1H-pyrazol-4-yl)-3-trifluoromethyl-benzamide (BF1)

The preparation took place in analogy to Example 72 (BD1), starting with 317 mg of 3-(trifluoromethyl)benzoyl chloride and stirring for 24 h before concentrating. Trituration of the residue with a solution of methanol/dichloromethane (5/95) gave the title compound as a white precipitate.
HPLC R_{T} 2.75 min
C₁₈H₁₄F₃N₃O (345.33) MS (ESI, method A) 346(M+H)

### b) 3-Methyl-5-(3-trifluoromethyl-phenyl)-1H-pyrazolo[4,3-c]isoquinoline, trifluoroacetic acid salt (BF)

The preparation took place in analogy to Example 73 (BE), using 305 mg of N-(3-methyl-5-phenyl-1H-pyrazol-4-yl)-3-trifluoromethyl-benzamide, except the residue was purified by HPLC (Monochrome 10u C18, 100x21.2mm column, water(0.1 % trifluoroacetic acid)/acetonitrile(0.1 % trifluoroacetic acid), gradient = 95/5 → 0/100) to give the title compound.
HPLC R_{T} 3.31 min
C₁₈H₁₂F₃N₃ (327.10) MS (ESI, method A) 328 (M+H)

### Reference Example 75

### 5-phenyl-3-trifluoromethyl-1H-pyrazolo[4,3-c]isoquinoline

### a) 4-Amino-5-phenyl-3-trifluoromethyl-1H-pyrazole (BG2)

A solution of 6.6 g of 4-nitroso-5-phenyl-3-trifluoromethyl-1H-pyrazole (prepared as in Saloutin et al, J. Flourine Chem, vol 84, pp107-111) in 200 mL ethanol was treated with 2.0 g 10% palladium/carbon catalyst and hydrogenated at RT and 50 psi for 7.5 hours. The reaction mixture was filtered through celite and washed well with ethanol. The filtrate was concentrated to give the title compound as a yellow solid.
C₁₀H₈F₃N₃ (227.20), MS (ESI, method B) 228(M+H).
TLC (1/1 ethyl acetate/heptane) R_{f}= 0.35.

### b) N-(5-Phenyl-3-trifluoromethyl-1H-pyrazol-4-yl)-benzamide (BG1)

A solution of 1.0 g of 4-amino-5-phenyl-3-trifluoromethyl-1H-pyrazole in 30 mL of dichloromethane was treated with 1.1 mL of pyridine followed by 0.57 mL of benzoyl chloride. The reaction mixture was stirred at RT for 5 hours and then concentrated. The residue was partitioned between ethyl acetate and water. The organic portion was washed with brine, dried over magnesium sulfate, filtered and concentrated. The residue was purified by MPLC, eluting with 2/3 ethyl acetate/heptane, to give the title compound as a white solid, melting point 229-230°C.
C₁₇H₁₂F₃N₃O (331.31), MS (ESI, method B) 330 (M-H).

### c) 5-Phenyl-3-trifluoromethyl-1H-pyrazolo[4,3-c]isoquinoline (BG).

A mixture of 1.02g of N-(5-phenyl-3-trifluoromethyl-1H-pyrazol-4-yl)-benzamide in 10 mL of nitrobenzene was treated with 0.41 mL of phosphorous oxychloride and stirred at 185°C for 6 h. The solvent was distilled off under high vacuum and the residue was partitioned between ethyl acetate and water. The organic portion was washed with saturated sodium bicarbonate, water and brine and dried over magnesium sulphate. Concentration and purification by MPLC, eluting with 1/3 ethyl acetate/heptane, gave the title compound as a white solid, melting point 264-265°C.
HPLC R_{T} 3.32 min
C₁₇H₁₀F₃N₃ (313.29), MS (ESI, method B) 314 (M+H).

### Example 76

### 5-(Pyridin-2-yl)-3-trifluoromethyl-1H-pyrazolo[4,3-c]isoquinoline

### a) N-(Pyridin-2-yl)-(5-phenyl-3-trifluoromethyl-1H-pyrazol-4-yl)amide (BH1)

A solution of 1.0 g of 4-amino-5-phenyl-3-trifluoromethyl-1H-pyrazole in 30 mL of dichloromethane was treated with 1.1 mL of pyridine followed by 0.94 g of picolyl chloride. The reaction mixture was stirred at RT for 24 hours and concentrated. The residue was partitioned between ethyl acetate and water. The organic portion was washed with brine, dried over magnesium sulfate, filtered and concentrated. The residue was purified by MPLC, eluting with 3/7 ethyl acetate/heptane, to give the title compound as a yellow semi- solid.
C₁₆H₁₁F₃N₄O (332.31), MS (ESI, method B) 331 (M-H)
TLC (1/1 ethyl acetate/heptane) R_{f} = 0.50.

### d) 5-(Pyridin-2-yl)-3-trifluoromethyl-1H-pyrazolo[4,3-c]isoquinoline (BH)

The preparation was carried out as in Example 75 (BG), using 1.02g of N-(pyridin-2-yl)-(5-phenyl-3-trifluoromethyl-1H-pyrazol-4-yl)-amide, except the reaction mixture was concentrated, then partitioned between ethyl acetate and water. The organic portion was washed with saturated sodium bicarbonate, water and brine, dried over magnesium sulphate and concentrated. The residue was purified by MPLC, eluting with 1/1ethyl acetate/heptane, giving the title compound as a beige solid.
HPLC R_{T} 2.77 min
C₁₆H₉F₃N₄ (314.29), MS (ESI, method B) 315 (M+H).

### Pharmacological Examples

### Example 77

The pyrazoloisoquinoline derivatives according to the invention were tested for inhibitory activity against NIK in various in vitro assay systems. In this connection, human peripheral blood lymphocytes were preincubated for 1 h with different concentrations of the compounds and then stimulated for 24 h with LPS or IL1 β. After that, a commercially obtainable ELISA test kit was used to measure the release of TNFα in the culture supernatant, and the IC50 for the given compound was determined. The cytotoxicity was measured by way of LDH release using a commercially obtainable test kit and the LD50 for the given compound was determined.

In another assay, heparinized whole human blood was preincubated for 1 h with different concentrations of the compounds and then stimulated with LPS for 24 h. Commercially available test kits were used to measure the release of IL1β, TNFα and IL6 in the supernatant after 24 h, and the IC50 for the given compound was determined.

The results are shown in the following tables 1, 2 and 3.

**Table 1: Inhibition of TNFα release in LPS-stimulated human peripheral blood lymphocytes:**

| Example No. | TNFα release IC50 (µM) | IL6 release IC50 (µM) | Cytotoxicity LD50 (µM) |
|---|---|---|---|
| 1 | 1.9 | 80 | > 100 |
| 2 | 9 | > 100 | > 100 |
| 3 | 7.5 | 80 | > 100 |

| | | | |
|---|---|---|---|
| The sign ">" denotes greater than | | | |

**Table 2: Inhibition of TNFα release in IL1 β-stimulated human peripheral blood lymphocytes:**

| Example No. | TNFα release IC50 (µM) | IL6 release IC50 (µM) | Cytotoxicity LD50 (µM) |
|---|---|---|---|
| 1 | 5 | 80 | >100 |
| 2 | 35 | > 100 | > 100 |
| 3 | 8 | > 100 | > 100 |

**Table 3: Inhibition of the release of IL1β, TNFα and IL6 in LPS-stimulated heparinized whole human blood:**

| Example No. | IL1β release IC50 (µM) | TNFα release IC50 (µM) | IL6 release IC50 (µM) |
|---|---|---|---|
| 1 | 33 | 12.5 | > 100 |
| 2 | 1.3 | 1.2 | 7 |
| 3 | 29 | 5 | > 100 |

### Example 78

### EAE Model

This example illustrates the efficay of the compounds of this invention in the treatment of multiple sclerosis. As described herein, experimental autoimmune encephalomyelitis (EAE) model is used to show such an effcicacy. The following procedures are employed in this model.

### Animals:

SJUJ female mice, 8 wks. old, are obtained from Jackson Laboratories.

### Antigens:

Myelin Proteolipid Protein (PLP 139-151) (HSLGKWLGHPDKF) (Cat # H-2478) is obtained from BACHEM, Bioscience, Inc., 3700 Horizon Dr., King of Prussia, PA 19406, 1-610-239-0300 (phone), 1-610-239-0800 (fax).

Complete Freund's Adjuvant H37 Ra [1 mg/ml Mycobacterium Tuberculosis H37 Ra] is obtained from Difco 1-800-521-0851 (Cat # 3114-60-5, 6X10ml).

Mycobacterium Tuberculosis is also obtained from Difco, 1-800-521-0851 (Cat # 3114-33-8, 6X100mg).

### Pertussis Toxin

Bordetella Pertussis, (Lyophilized powder containing PBS and lactose) is obtainde from List Biological Laboratories, 1-408-866-6363 (Product #180, 50ug @ $140.00ea.).

### Induction of EAE in Mice

PLP139-151 peptide is dissolved in H₂O:PBS (1:1) solution to a concentration 7.5 mg/10ml (for 75 ug PLP per group) and emulsified with an equal volume of CFA supplemented with 40mg/10ml heated-killed mycobacterium tuberculosis H37Ra. Mice are injected s.c. with 0.2 ml of peptide emulsion in the abdominal flank (0.1 ml on each side). On the same day and 72 hours later, mice are injected i.v. with 100□l of 35 ng and 50 ng of Bordetella Pertussis toxin in saline respectively.

### Clinical Assessment

*STAGE 0: Normal*
*STAGE 0.5: Partial limp tail*
*STAGE 1: Complete Limp Tail*
*STAGE 2: Impaired righting reflex*
*STAGE 2.5: Righting reflex is delayed (Not weak enough to be stage 3).*
*STAGE 3: Partial hind limb paralysis*
*STAGE 3.5: One leg is completely paralyzed, and one leg is partially paralyzed,*
*STAGE 4: Complete hind limb paralysis*
*STAGE 4.5: Legs are completely paralyzed and Moribund*
*STAGE 5: Death due to EAE*

### Clinical courses of EAE

*Acute phase:* First clinical episode (Day 10-18)
*Remission:* Phase of clinical improvement following a clinical episode; characterized by a reduction (>= one grade) in clinical score for at least two days after the peak score of acute phase or a disease relapse.
*Relapse:* Increase of at least one grade in clinical score for at least two days after remission has been attained.

The animals treated with the compounds of this invention generally would be expected to show improvements in clinical scores.

### Example 79

This Example illustrates the efficacy of the compounds of this invention by way of inhibiting NIK-mediated release of IL-1β from human macrophages activated by the Alzheimer's beta amyloid peptide 1-42.

Cell isolation: Monocytes are isolated from peripheral blood mononuclear cells (PBMCs) as follows. Whole blood is layered directly onto Histopak 1077-1 columns (Sigma Biochemicals) and centrifuged at 800 x g for 15 minutes. The PBMC band of cells is removed to a fresh 50 ml culture tube and diluted 1:1 with wash buffer (Phosphate buffered saline, pH 7.4 containing 2 mM EDTA and 5 mg/ml BSA) followed by centrifugation at 800 x g for 5 minutes. Cells are then washed by sequential resuspension of the cell pellet in wash buffer and centrifugation at 600 x g for 5 minutes. The wash process is repeated until the supernatent is clear of contaminating platelets (5 to 6 washes). Monocytes are then purified from the PBMCs by negative selection using a monocyte isolation kit (Miltenyi Biotec, Inc) that contains antibodies to non-monocytic cells, running the cells over a magnetic column to remove antibody-bound cells, and collecting the flow through volume of monocytes. Monocytes are washed once with wash buffer and seeded at 10E5 cells per well in 100 µl serum-free RPMI 1640 in 96-well plates and incubated for 1 hour at 37°C in a 5% CO₂/95% humidified tissue culture incubator. After 1 hour, the medium is replaced with 100 µl complete culture medium (RPMI 1640, 10% human serum-type AB (heat inactivated), 25 mM HEPES, 2 mM glutamine, 50 U/ml each of penicillin and streptomycin) and incubated overnight (16 hours).

Dosing regimen: The next day, the culture medium is replaced with 100 µl fresh complete culture medium in the absence or presence of human beta amyloid 1-42 peptide (5 µM) and incubated at 37°C in a 5% CO₂/95% humidified tissue culture incubator for 5 hours. Medium is then removed and discarded. Each well is washed once with Hanks buffered saline (HBSS) containing 1 mM CaCl₂ followed by the addition of 80 µl of HBSS/CaCl₂. Samples are then given either 10 µl of HBSS/CaCl₂ or 10 µl of the NIK inhibiting compound of the present invention (10 x stock in HBSS/CaCl₂ for a final concentration of 23 nM and 206 nM) and incubated 15 minutes in the tissue culture incubator followed by the addition of either 10 µl of HBSS/CaCl₂ or 10 µl of benzoylbenzoyl ATP (BzATP; 3 mM stock in HBSS/CaCl₂ for a 300 µM final concentration) and incubated for a further 30 minutes in the tissue culture incubator. Medium is then removed to new 96-well plates for storage at - 70°C until the IL-1β content is quantitated by ELISA (from R&D Systems). The cells are washed once with HBSS/CaCl₂ followed by lysing the cells with 100 µl ice cold lysis buffer (100 mM Tris, pH 7.6, 1 % triton X-100, and 1 tablet per 30 ml Complete TM protease inhibitor from Roche Biochemicals, Inc). Cell lysates are stored at -70°C until the IL-1β is quantitated by ELISA.

The compounds of this invention generally would be expected to show decrease in the BzATP-induced IL-1β secretion.

## Claims

1. A compound selected from the group consisting of:
5-pyridin-2-yl-3-trifluoromethyl-1H-pyrazolo[4,3-c]isoquinoline,
3-methyl-5-(2-trifluoromethyl-phenyl)-1H-pyrazolo[4,3-c]isoquinoline,
3-methyl-5-(3-trifluoromethyl-phenyl)-1H-pyrazolo[4,3-c]isoquinoline,
3-methyl-5-(4-trifluoromethyl-phenyl)-1H-pyrazolo[4,3-c]isoquinoline,
1,3-dimethyl-5-(3-trifluoromethylphenyl)-1H-pyrazolo[4,3-c]-isoquinoline,
1,3-dimethyl-5-(2,6-difluorophenyl)-1H-pyrazolo[4,3-c]-isoquinoline,
5-methoxymethyl-3-methyl-1H-pyrazolo[4,3-c]-isoquinoline,
7-methoxycarbonyl-3-methyl-5-phenyl-1H-pyrazolo[4,3-c]-isoquinoline,
7-methoxycarbonyl-3-methyl-5-pyridin-2-yl-1H-pyrazolo[4,3-c]-isoquinoline,
7-dimethylamino-3-methyl-5-phenyl-1H-pyrazolo[4,3-c]isoquinoline,
7-dimethylamino-3-methyl-5-pyridin-2-yl-1H-pyrazolo[4,3-c]-isoquinoline,
1,3-dimethyl-5-(3-methyl-thiophen-2-yl)-1H-pyrazolo[4,3-c]-isoquinoline,
3-methyl-5-phenyl-9-trifluoromethyl-1H-pyrazolo[4,3-c]isoquinoline,
3-methyl-5-pyridin-2-yl-9-trifluoromethyl-1H-pyrazolo[4,3-c]-isoquinoline, and
3-methyl-5-(2,3,4,5,6-pentafluoro-phenyl)-1H-pyrazolo[4,3-c]-isoquinoline.

2. A pharmaceutical composition comprising a therapeutically effective content of at least one compound as claimed in claim 1 together with a pharmaceutically suitable carrier.

3. The use of a compound according to claim 1 for producing a medicament for treating a disease condition selected from the group consisting of osteoarthritis, rheumatoid arthritis and asthma.

4. The use as claimed in claim 3, wherein the disease condition is osteoarthritis.

5. The use as claimed in claim 3, wherein the disease condition is rheumatoid arthritis.

## Patentansprüche

1. Verbindungen ausgewählt aus der Gruppe bestehend aus:
5-Pyridin-2-yl-3-trifluormethyl-1H-pyrazolo[4,3-c]isochinolin,
3-Methyl-5-(2-trifluormethylphenyl)-1H-pyrazolo[4,3-c]isochinolin,
3-Methyl-5-(3-trifluormethylphenyl)-1H-pyrazolo[4,3-c]isochinolin,
3-Methyl-5-(4-trifluormethylphenyl)-1H-pyrazolo[4,3-c]isochinolin,
1,3-Dimethyl-5-(3-trifluormethylphenyl)-1H-pyrazolo[4,3-c]isoquinolin,
1,3-Dimethyl-5-(2,6-difluorphenyl)-1H-pyrazolo[4,3-c]isoquinolin,
5-Methoxymethyl-3-methyl-1H-pyrazolo[4,3-c]isochinolin,
7-Methoxycarbonyl-3-methyl-5-phenyl-1H-pyrazolo[4,3-c]isochinolin,
7-Methoxycarbonyl-3-methyl-5-pyridin-2-yl-1H-pyrazolo[4,3-c]isochinolin,
7-Dimethylamino-3-methyl-5-phenyl-1H-pyrazolo[4,3-c]isochinolin,
7-Dimethylamino-3-methyl-5-pyridin-2-yl-1H-pyrazolo[4,3-c]isochinolin,
1,3-Dimethyl-5-(3-methylthiophen-2-yl)-1H-pyrazolo[4,3-c]isochinolin,
3-Methyl-5-phenyl-9-trifluormethyl-1H-pyrazolo[4,3-c]isochinolin,
3-Methyl-5-pyridin-2-yl-9-trifluormethyl-1H-pyrazolo[4,3-c]isochinolin und
3-Methyl-5-(2,3,4,5,6-pentafluorphenyl)-1H-pyrazolo[4,3-c]isochinolin.

2. Pharmazeutische Zusammensetzung, enthaltend eine therapeutisch wirksame Menge wenigstens einer Verbindung nach Anspruch 1 zusammen mit einem pharmazeutisch unbedenklichen Träger.

3. Verwendung einer Verbindung nach Anspruch 1 zur Herstellung eines Medikaments zur Behandlung von Krankheiten ausgewählt aus der Gruppe bestehend aus Osteoarthritis, rheumatoider Arthritis und Asthma.

4. Verwendung nach Anspruch 3, wobei es sich bei der Krankheit um Osteoarthritis handelt.

5. Verwendung nach Anspruch 3, wobei es sich bei der Krankheit um rheumatoide Arthritis handelt.

## Revendications

1. Composé choisi dans le groupe constitué par : la 5-pyridin-2-yl-3-trifluorométhyl-1H-pyrazolo[4,3-c]isoquinoléine, la 3-méthyl-5-(2-trifluorométhylphényl)-1H-pyrazolo[4,3-c]isoquinoléine, la 3-méthyl-5-(3-trifluorométhyl-phényl)-1H-pyrazolo[4,3-c]isoquinoléine, la 3-méthyl-5-(4-trifluorométhylphényl)-1H-pyrazolo[4,3-c]isoquinoléine, la 1,3-diméthyl-5-(3-trifluorométhylphényl)-1H-pyrazolo[4,3-c]-isoquinoléine, la 1,3-diméthyl-5-(2,6-difluorophényl)-1H-pyrazolo[4,3-c]-isoquinoléine, la 5-méthoxyméthyl-3-méthyl-1H-pyrazolo[4,3-c]-isoquinoléine, la 7-méthoxycarbonyl-3-méthyl-5-phényl-1H-pyrazolo[4,3-c]-isoquinoléine, la 7-méthoxycarbonyl-3-méthyl-5-pyridin-2-yl-1H-pyrazolo[4,3-c]-isoquinoléine, la 7-diméthylamino-3-méthyl-5-phényl-1H-pyrazolo[4,3-c]isoquinoléine, la 7-diméthylamino-3-méthyl-5-pyridin-2-yl-1H-pyrazolo[4,3-c]-isoquinoléine, la 1,3-diméthyl-5-(3-méthyl-thiophén-2-yl)-1H-pyrazolo[4,3-c]-isoquinoléine, la 3-méthyl-5-phényl-9-trifluorométhyl-1H-pyrazolo[4,3-c]isoquinoléine, la 3-méthyl-5-pyridin-2-yl-9-trifluorométhyl-1H-pyrazolo[4,3-c]-isoquinoléine, et la 3-méthyl-5-(2,3,4,5,6-pentafluoro-phényl)-1H-pyrazolo[4,3-c]-isoquinoléine.

2. Composition pharmaceutique contenant une teneur thérapeutiquement efficace d'au moins un composé selon la revendication 1 avec un support pharmaceutiquement approprié.

3. Utilisation d'un composé selon la revendication 1 pour produire un médicament destiné au traitement d'un état de maladie choisi dans le groupe constitué par l'ostéoarthrite, l'arthrite rhumatoïde et l'asthme.

4. Utilisation selon la revendication 3, où l'état de maladie est l'ostéoarthrite.

5. Utilisation selon la revendication 3, où l'état de maladie est l'arthrite rhumatoïde.
